(19) Europäisches Patentamt

European Patent Office

Office européen des brevets

(11) Veröffentlichungsnummer : **0 331 624 B1**

# (12) EUROPÄISCHE PATENTSCHRIFT

(45) Veröffentlichungstag der Patentschrift :
28.08.91 Patentblatt 91/35

(51) Int. Cl.⁵ : **A61F 2/38**

(21) Anmeldenummer : 89810032.6

(22) Anmeldetag : 16.01.89

(54) **Metallenes Knochenimplantat.**

(30) Priorität : 29.02.88 CH 747/88

(43) Veröffentlichungstag der Anmeldung :
06.09.89 Patentblatt 89/36

(45) Bekanntmachung des Hinweises auf die
Patenterteilung :
28.08.91 Patentblatt 91/35

(84) Benannte Vertragsstaaten :
**AT BE DE ES FR GB IT NL SE**

(56) Entgegenhaltungen :
EP-A- 0 176 728
FR-A- 2 478 462
FR-A- 2 521 421
US-A- 3 878 566

(73) Patentinhaber : **GEBRÜDER SULZER
AKTIENGESELLSCHAFT
Zürcherstrasse 9
CH-8401 Winterthur (CH)**

(72) Erfinder : **Frey, Otto, Dr.
Wallrütistrasse 56
CH-8400 Winterthur (CH)**
Erfinder : **Scheier, Heinrich, Prof. Dr.-med.
Neumünsterallee 3
CH-8008 Zürich (CH)**

EP 0 331 624 B1

**Beschreibung**

Die Erfindung betrifft ein metallenes Knochenimplantat, das auf einer Plateaufläche eines Knochens mit Hilfe von Knochenzement verankerbar ist und eine gegen die Plateaufläche gerichtete, erhöhte äussere Begrenzung aufweist.

Knochenimplantate der vorstehend genannten Art, die beispielsweise als Tibiateile oder Femurschalen von Kniegelenkprothesen dienen, sind bekannt. Als Beispiel für die Vielzahl der Konstruktionen sei die CH-PS 632151 genannt.

Bei der Implantation derartiger Implantate haben sich Schwierigkeiten ergeben, da beim Eintreiben des Implantats in den Knochen Knochenzement verdrängt wird und seitlich zwischen Implantat und Knochen herausquillt. Dadurch ist eine gleichmässige Mindestdicke des Zementbettes, die für eine einwandfreie Verankerung des Implantats notwendig ist, nicht mehr gewährleistet und die Arbeit des Operateurs erschwert.

Aus der EP-A-0176728 ist eine Bandscheibenprothese bekannt, deren zu einem Wirbel gerichteter Rand erhöht und mit einzelnen, auf ihm verteilten, scharfen Zähnen versehen ist. Durch diese Zähne können weder ein Herausquellen des Knochenzementes verhindert noch eine scharfe Abtrennung des ausgetretenen Zements erreicht werden.

Bei einer Patella-Prothese nach dem US-Patent 3,878,566 ist der Femurteil mit einem erhöhten Rand versehen, der ebenfalls gegen den Knochen gerichtet ist. Durch diesen Rand wird zwar ein Teil des Knochenzementes am Herausquellen gehindert, eine scharfe Abtrennung dieses überschüssigen Zementes ist jedoch nicht möglich.

Aufgabe der Erfindung ist es, ein auf einer Plateaufläche mit Hilfe von Knochenzement zu verankerndes Implantat zu schaffen, bei dem nicht nur die erforderliche Mindestdicke des Knochenzementbettes gewährleistet ist, sondern auch der überschüssige Zement einfach und sauber entfernt werden kann. Diese Aufgabe wird dadurch gelöst, dass diese äussere Begrenzung des Implantates mindestens über weite Teile ihrer Umfangslänge als messerartige Schneidkante ausgebildet ist. Die Schneidkante, die mit ihrer Höhe von 1-3 mm ein vollständiges Herausquellen des Knochenzementes verhindert und einen Teil davon gefangen hält, ermöglicht, dass der herausgequollene Zement glatt abgeschnitten wird und daher leicht und sauber entfernt werden kann.

Im folgenden wird die Erfindung anhand von Ausführungsbeispielen näher erläutert.

Fig. 1    zeigt in einer Ansicht von vorn ein Kniegelenk, in das eine unikompartmentale Prothese eingesetzt ist, wobei die Femurschale teilweise im Schnitt dargestellt ist, während der Tibiateil nur angedeutet wiedergegeben ist ;

Fig. 2    ist ein Schnitt durch einen Tibiateil einer bikondilären Kniegelenkprothese, während

Fig. 3    einen Schnitt durch den zu Fig. 2 gehörigen Femurteil darstellt.

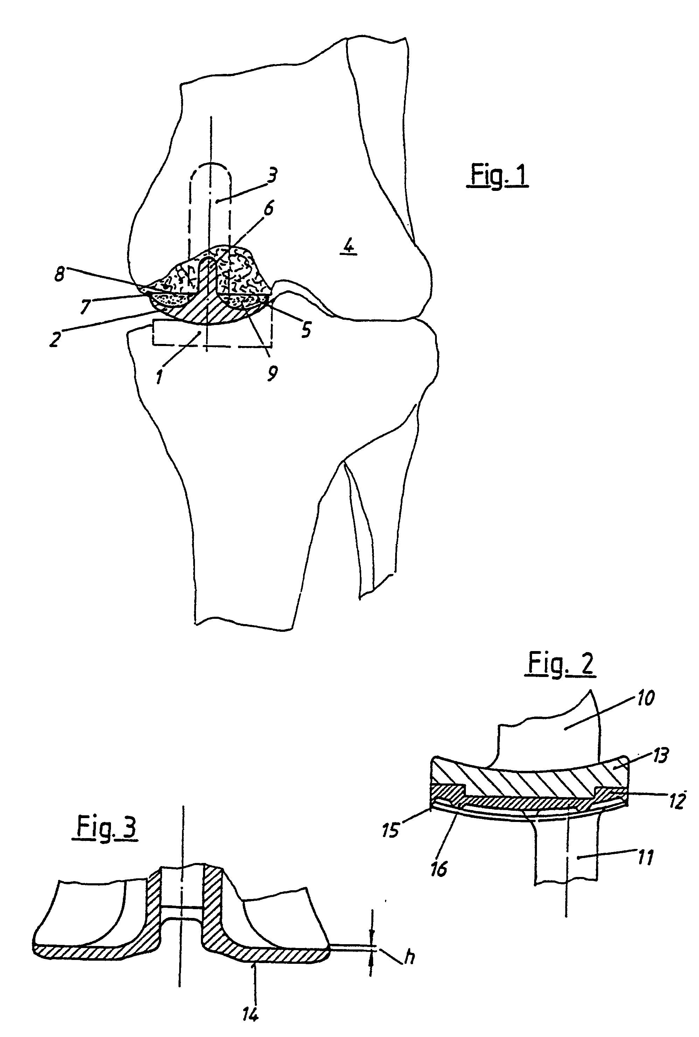

Bei dem in der Fig. 1 gezeigten linksseitigen Kniegelenk ist die mediale Kondyle durch eine unikompartmentale Prothese ersetzt, deren Tibiateil 1 nur schematisch angedeutet ist. Die einer Femurkondyle nachgeformte Femurschale 2 ist mit Zapfen 3, von denen nur einer dargestellt ist, im Femur 4 mit Hilfe eines Knochenzementbettes 5 verankert. In Richtung anterior/posterior erstreckt sich zwischen den Zapfen 3 eine Verstärkungsrippe 6, durch die vor allem die Stabilität der Femurschale 2 erhöht wird.

Gemäss der vorliegenden Erfindung ist der äussere Rand der Femurschale mit einer scharfen Schneidkante 7 versehen, die längs des ganzen Umfanges der Schale 2 verläuft. Beim Einschlagen des Implantates in den Knochen 4, das sich über das Zementbett 5 auf der operativ vorbereiteten plateauartigen Schnittfläche 8 des Femurs 4 abstützt, hält die Kante 7 einerseits einen Teil des Knochenzementes 5 gefangen und schneidet andererseits in einer glatten Schnittfläche den überschüssigen herausgequollenen Zement 5 ab.

Da die Rückseite der Femurschale 2 beidseits der Rippe 6 muldenförmig verläuft, ist die Höhe der Schneidkante 7 in diesem Ausführungsbeispiel der Erfindung von untergeordneter Bedeutung. Denn in den Mulden 9 ist die erforderliche Mindestdicke für den Knochenzement 5 jederzeit gewährleistet.

Der Tibiateil 10 (Fig. 2) einer bikondylären Knieprothese, der mit einem Schaft 11 in der nicht dargestellten Tibia verankerbar ist, trägt auf einer Platte 12 einen Kunststoffteil 13, der als Lagerfläche für die Femurkondylen 14 (Fig. 3) dient. Das der Tibia zugewandte Plateau ist wiederum von einer scharfen Kante 15 umrandet ; weiterhin sind auf der Plateaufläche Rippen 16 als zusätzliche Oberflächenstrukturelemente vorgesehen. Die Höhe der Kante 15 beträgt gemessen vom Boden des Plateaus etwa 2 mm.

Die Femurkondylen 14 des zum Tibiateil 10 nach Fig. 2 gehörenden Femurteils schliesslich sind auf der dem Knochen zugewandten Innenseite längs ihres äusseren Randes ebenfalls von einer scharfen Kante 15 eingefasst, deren Höhe mit h bezeichnet ist und etwa 1 mm beträgt.

**Patentansprüche**

1. Metallenes Knochenimplantat (2, 12), das auf einer Plateaufläche (8) eines Knochens (4) mit Hilfe von Knochenzement (5) verankerbar ist und eine gegen die Plateaufläche (8) gerichtete, erhöhte äussere Begrenzung aufweist, dadurch gekennzeichnet, dass diese äussere Begrenzung des Implantates (2) mindestens über weite Teile ihrer Umfangslänge als messerartige Schneidkante (7) ausgebildet ist.

2. Knochenimplantat nach Anspruch 1, dadurch gekennzeichnet, dass die Höhe (h) der Schneidkante (7) 1 bis 3 mm beträgt.

**Claims**

1. A metal bone implant (2, 12) adapted to be anchored on a plateau surface (8) of a bone (4) by means of bone cement (5) and having a raised outer boundary directed towards the plateau surface (8), characterised in that said outer boundary of the implant (2) is constructed as a cutting edge (7) after the style of a knife over at least considerable parts of its peripheral length.

2. A bone implant according to claim 1, characterised in that the height (h) of the cutting edge (7) is 1 to 3 mm.

**Revendications**

1. Implant métallique pour os (2, 12) qui peut être ancré sur une surface de plateau (8) d'un os (4) à l'aide de ciment pour os (5) et qui présente une délimitation extérieure surélevée, orientée vers la surface de plateau (8), caractérisé en ce que cette délimitation extérieure de l'implant (2) se présente, sur au moins de grandes parties de sa longueur périphérique, sous la forme d'une arête de coupe (7) en lame.

2. Implant pour os selon la revendication 1, caractérisé en ce que la hauteur (h) de l'arête de coupe (7) est comprise entre 1 et 3 mm.

Fig. 1

Fig. 2

Fig. 3